# EUROPEAN PATENT SPECIFICATION

(11) **EP 2 186 915 B1**
(45) Date of publication and mention of the grant of the patent: **28.09.2011**
(21) Application number: 08168537.2
(22) Date of filing: 06.11.2008
(51) Int. Cl.: C12Q 1/68

(54) **Method and kit for detecting genetic predisposition for crooked tail syndrome (CTS) in bovine individuals**
Verfahren und Kit zur Erkennung der genetischen Veranlagung für das CTS in bovinen Individuen
Procédé et kit pour la détection de prédisposition génétique au syndrome du canal carpien (CTS) sur les bovins

(43) Date of publication of application: 19.05.2010
(73) Proprietor: Université de Liège, 4031 Angleur (BE)
(72) Inventor: Georges, Michel, Prof., 4000 Liege (BE); Charlier, Carole, Dr., 4000 Liege (BE)

(56) References cited:
- CHARLIER CAROLE ET AL: "Highly effective SNP-based association mapping and management of recessive defects in livestock" NATURE GENETICS, NATURE PUBLISHING GROUP, NEW YORK, US, vol. 40, no. 4, 1 April 2008 (2008-04-01), pages 449-454, XP002507256 ISSN: 1061-4036 [retrieved on 2008-03-16]
- DATABASE EMBL [Online] 19 March 2008 (2008-03-19), "Bos taurus clone CH240-283B3, WORKING DRAFT SEQUENCE, 8 unordered pieces." XP002518831 retrieved from EBI accession no. EMBL:AC218425 Database accession no. AC218425
- DATABASE GENBANK [Online] 29 July 2008 (2008-07-29), "PREDICTED: Bos taurus similar to mannose receptor, C type 2 (MRC2), mRNA" XP002518832 Database accession no. XM_607489
- ENGELHOLM L H ET AL: "The urikonase receptor associated protein (uPARAP/Endo180): A novel internalization receptor connected to the plasminogen activation system" TRENDS IN CARDIOVASCULAR MEDICINE, ELSEVIER SCIENCE, NEW YORK, NY, US, vol. 11, no. 1, 1 January 2001 (2001-01-01), pages 7-13, XP002976375 ISSN: 1050-1738

### SEQUENCE LISTING

<110> Université de Liège
<120> Method and kit for detecting genetic predisposition for crooked Tail syndrome (CTS) in bovine individuals
<130> 2008-16
<160> 10
<170> PatentIn version 3.3
<210> 1
   <211> 59734
   <212> DNA
   <213> Bos sp.
<220>
   <221> gene
   <222> (1)..(59734)
   <223> wild-type MRC2 genomic sequence
<220>
   <221> misc_feature
   <222> (42418)..(42467)
   <223> n is a, c, g, or t
<400> 1
<210> 2
   <211> 59732
   <212> DNA
   <213> Bos sp.
<220>
   <221> gene
   <222> (1)..(59732)
   <223> mutated MRC2 genomic sequence
<220>
   <221> misc_feature
   <222> (42418)..(42467)
   <223> n is a, c, g, or t
<400> 2
<210> 3
   <211> 6172
   <212> DNA
   <213> Bos sp.
<220>
   <221> gene
   <222> (1)..(6172)
   <223> wild-type MRC2 cDNA sequence
<400> 3
   gcgctccctc ggtccccgca tcccccgcgc gtcacgccgc gggcatgggg cccggccggc 60
   cggccctcgc gccctggcct catcacctgc tgcgatgcgc cttgctcttc gggggtctgc 120
   acttcggccg cccggccgcc gccgccgccg ccgccctccc ggagcccaac gtcttcgtca 180
   tcttcagcca tggactgcag ggctgcctgg aggcccaggg tgggcaagtc cgagtctctc 240
   cagcctgcaa tgccagcctt cctgcccagc gctggaagtg ggtctcccga aaccggctct 300
   tcaacctggg caccatgcag tgcctgggca cgggctggcc gggcaccaac accaccgcct 360
   ccctgggcat gtacgagtgt gaccgcgagg cactgagtct tcgctggaac tgtcgcaccc 420
   tgggcgacca gctgtccttg ctcctggggg gccgtgtcgg caacacatcc aaggctggca 480
   gccccgagcg cggtgaccag acccgcagcg gccagtggcg catctatggc agcgatgagg 540
   acctgtgtgc gcggccctac tatgaggtct acaccatcca gggcaactcc cacgggaagc 600
   cgtgcaccat ccccttcaaa tatgacaacc agtggttcca tggctgcacc agcactggcc 660
   gcgaggatgg gcacctgtgg tgcgccacca cccaggacta cggcaaggac gagcgctggg 720
   gcttctgccc catcaagggt aacgactgcg agaccttctg ggacaaggac cagctgaccg 780
   acagctgcta tcagttcaac ttccagtcca cgctgtcctg gagggaggcc tgggccagct 840
   gcgagcagca gggggcagac ctgctgagca tcacggagat ccacgagcag acctacatca 900
   atgggctcct cactggctac agctccacac tgtggattgg ccttaacgac ctggacacca 960
   gtggaggctg gcagtggtca gacaactcgc ctctcaagta cctcaactgg gagagtgatc 1020
   agccggacaa cccgagcgag gagaactgcg gggtgatccg cacggagtcg tccggcggct 1080
   ggcagaaccg cgactgcagc atcgcgctgc cctacgtctg caagaagaag cccaacgcca 1140
   cggcctccga gcccccacct cccgatgtgt gggccaacgt gaaggtggag tgtgagccca 1200
   gctggcagcc cttccagggc cactgctacc gcctgcaggc tgagaagcgc agctggcagg 1260
   agtccaagaa gatgtgtctg cggggtgggg gcgacttgct cagcatccac agcatggcgg 1320
   agctggagtt catcaccaag cagatcaagc aggaggtgga ggagctgtgg attggtctca 1380
   acgacctgaa actgcagatg aattttgagt ggtccgacgg gagcctcgtg agcttcaccc 1440
   attggcaccc ctttgagccc aacaacttcc gagacagcct ggaggactgt gtcaccatct 1500
   gggggccgga aggtcgctgg aacgacagtc cctgtaacca gtccctgccg tccatttgca 1560
   agaaggcagg ccagctgagc cagggggctg ccgaggagga ccacggctgc cggaagggtt 1620
   ggacgtggca cagcccctcc tgctactggc tgggagagga ccaggtgacc tacagtgagg 1680
   cccggcgcct gtgcaccgac cacggctctc agctggtcac catcaccaac aggttcgagc 1740
   aggcctttgt cagcagcctc atctacaact gggatggcga gtacttctgg acggccctgc 1800
   aggacctcaa cttcaccggt tccttccgct ggctcagcgg ggacgaggtc atgtacaccc 1860
   actggaaccg ggaccagcct gggtacagcc gtgggggctg cgtggccctg gccacaggca 1920
   gtgccatggg gctgtgggag gtgaagaact gcacatcgtt ccgggctcgc tacatctgcc 1980
   gccagagcct gggcacgccc gtgacgcctg agctgcctgg gctagacccc acgcccagcc 2040
   tcaccggcgc ctgtccgcag ggctggggct cagatcccaa actccggcac tgctataagg 2100
   tgttcagctc ggagcggctg caggataaga agacctgggt ccaggcccag ggggcctgcc 2160
   aggagctggg ggcccagctg ctgagcctgg ccagctacga agaggagcac tttgtggcca 2220
   acatgctcaa caagatcttc ggtgaatcag agcccgagat ccacgagcag cactggttct 2280
   ggatcggcct gaaccgccga gaccccggag ccgggcagag ctggcgctgg agtgatggac 2340
   ttgggttctc ttaccacaat ttcgaccgga gccggcacga cgatgatgac atccgcagct 2400
   gcacggtgct cgacttggcc tccctgcagt gggtggctat gcagtgcgag acgcagctgg 2460
   actggatctg caagatccct cgaggcacgg acgtgcggga gcctgacgtc agcccgcaag 2520
   gccggcggga atggctgcgt ttccaggagg ctgagtataa gttcttcgag caccactcca 2580
   cgtgggcgca ggcgcagcgc atctgcacgt ggttccaggc tgaactggtc tcagtgcaca 2640
   gccaggccga gctggacttc ctggggcaca acctgcagaa gttctctcgg ggccaggagc 2700
   agcactggtg gatcggcctg cacacctcag agagcgacgg gcgcttcagg tggacagatg 2760
   gttccattat aaacttcatc tcctgggccc ccggcaaacc tcggcccatc ggcaaggaca 2820
   agaagtgcgt gtacatgatg gccagccgag aggactgggg ggaccagagg tgcacaacag 2880
   ccttgcctta catctgcaag cggcgcaaca gcaccagaga gcagcagccc ccagacctgc 2940
   cgcccacagg gggctgcccc tctggctgga gccagttcct gaacaagtgt ttcagaatcc 3000
   aaggccagga cccccaggac cgggtgaagt ggtcagaggc acagttctcc tgtgaacagc 3060
   aagaggccca actggtcacc attgcaaacc ccttagagca agcgttcatc acagccagcc 3120
   tgcccaatgt gacctttgac ctttggattg gcctccatgc ctcgaagagg gacttccagt 3180
   gggtggagca ggagcctctg cagtacgcca actgggcccc tggggagccc tctggcccta 3240
   gccctgctcc cagcggcaac ataccgacca gctgtgccgt ggtcctgcat agcccctcaa 3300
   cccacttcac tggccgctgg gatgaccgga gctgtgcaga ggagacccat ggcttcatct 3360
   gccagaaggc cacggacccc tccctgagcc catccccagc agcgtcgccc cctgccccgg 3420
   gcgctgagct ctcctacctc aacggcacct tccggctgct gcagaagccg ctgcgctggc 3480
   acgacgccct gttgctgtgt gagagccgaa atgccagcct ggcgcacgtg cttgacccct 3540
   acacccaggc cttcctcact caggccgccc gagggctgcg cacgccactc tggattgggc 3600
   tggccagtga ggagggctcc cggcggtact cttgggtctc ggaggagccg ctgagctacg 3660
   tgagctggca ggacggggag ccccagcacc ccgggggctg cgcctacgtg gacacggatg 3720
   gtgcctggcg caccaccagc tgtgacacca agttgcaggg tgctgtgtgt ggagttcacg 3780
   gcgggccccc tcctccgcga cgaataagct accacgggag ctgtccccag gggctggcgg 3840
   actcggcatg gattcccttc cgagagcact gctactcctt ccatatggag ctgctgctgg 3900
   gccacaagga ggcgctgcag cgctgccaga gagcgggcgg cgcggtcctg tccatcctgg 3960
   atgagatgga gaacgtgttt gtctgggagc atctgcagag ctctgagggc cagagtcggg 4020
   gcgcctggct gggcatgaac ttcaacccca aggggggcac cctggtctgg caggacaaca 4080
   cagctgtgaa ctactccaac tgggggcccc cgggcctggg ccccagcatg ctgagccaca 4140
   acagctgcta ctggatccag agcagcagcg ggctgtggcg cccgggggcc tgcaccaaca 4200
   tcagcatggg cgtcgtctgt aagctccctc gagctgaggc gagcagcttc tccccatcag 4260
   ccctcccgga gaaccccgcg gccctggtgg tggtgctgat ggcggtgctc ctgctcctgg 4320
   ctctgctgac ggctgccctg attctctacc ggcggcgaca gagcgtcgag cgtggggcct 4380
   tcgagggtgc ccgctacagc cgcagctcct ccggccccag cgaggccacc gagaagaaca 4440
   tcctggtgtc cgacatggaa atgaacgagc agcaggagta gagccgaggg cgtgggcggg 4500
   gccgagggcg tgggcggggc cgggggagct gggcgcccca ccagctgcgg tccagtcggc 4560
   ctatgggggg aggtggcgcc ctggggagcg ctgtggggag ctgaggctgg cagggcctgg 4620
   gctggtgggg tccttccctc ccatgagggc tgggctgagg cctggctgag tgcagcatgg 4680
   cgtttccttt tgggggggcc ctaaggtctt gtcacccggg cctgtacccc catggtcacc 4740
   ggaggcagga caggagcctc tttctccctg cccccacccc ccaggcctgt cccccaaccc 4800
   tgggaccccc tcttctcgct gcaaagtctg aggagcctcc ctgagccctc agccggcctc 4860
   tgtcgcttct cccctaccct ggcagctcag ctcctcgtcc cacatcctcc ctgcccccca 4920
   cccgctgtcc gcccgccctg catcctgcct gctctcccag cccttccttc tgagccaggg 4980
   ccctggggtc tggggagccc tctcttgctg ttggtggggg tcacagggtg agcatttgtc 5040
   aattgtgtgc ctgctggggt ggccgtaggg catgacggga gggttctggg ctgggactga 5100
   ggaccggagc atcactgtgg tatcagctgg gctggagaca ggtccgggga gagacaccct 5160
   gacctcctgg ggatgaccgg gctgggctgg ggtgtcatct cctgccggtc agaggagggc 5220
   tctgtccctg taaagtcccc tgtggggacc aaagtaagtt ccctaaggtt ttcaggtcct 5280
   ggctgtagtt tggagagaga gaaggtggtt ggccagggtc agtgggagct ggggtgcgcc 5340
   ctctaagggg ggcccagagg agcagcacct gaaccccctg gagtggcccc tgtgtggggg 5400
   agacgaccca cccagaacct ttccaagagg ccggagcccc agttagaatc accagtcctg 5460
   gcgccatggc ccagggcaat gtgagcacag actccaagac acggtccttt ttgtagttct 5520
   taattttttt taatgtgcca ttattgtttt aaaaagaaaa agataaaaga aaagcaaaca 5580
   aataaaacac ctttcagagg cttgagagag aagggtcccc actttgtttt gttgggagtc 5640
   ttgtccttca tactgcttca aagatacatg ttattttggg gggcttgcat ctgttttgct 5700
   tctgtcttgg ggccacggga ccccagcctt ctctctgctc ctggggatac gtgtgagagt 5760
   aagcattcag gagccaaagg acagtggcgt gagcttgggc aacttggctc ccctttcaaa 5820
   gtgtaaaaca ggagaacagg atgcacctcc cgagctcctt taaggaggtt gaggattaag 5880
   tgagatcacc tgggcaaagc aatctgcaca gggccttggc gctcgagctg taattgtccc 5940
   ataaacgtca gctggtactg cttgtctctg ggctgtggct cccaggccag cgtgtgaggt 6000
   acccctgcca gaccgcggtg ggggccccac accaccatgc ttccaagaca gacctgcatt 6060
   tgagctgcaa gacagtgagt acacatttgt ccctgggtag tgtgggctct gaaactgccc 6120
   tgaggcaggg gagggggtca caggaccccg cccaccacca tcacctccat tc 6172
<210> 4
   <211> 6170
   <212> DNA
   <213> Bos sp.
<220>
   <221> gene
   <222> (1)..(6170)
   <223> mutated MRC2 cDNA sequence
<400> 4
   gcgctccctc ggtccccgca tcccccgcgc gtcacgccgc gggcatgggg cccggccggc      60
   cggccctcgc gccctggcct catcacctgc tgcgatgcgc cttgctcttc gggggtctgc     120
   acttcggccg cccggccgcc gccgccgccg ccgccctccc ggagcccaac gtcttcgtca     180
   tcttcagcca tggactgcag ggctgcctgg aggcccaggg tgggcaagtc cgagtctctc     240
   cagcctgcaa tgccagcctt cctgcccagc gctggaagtg ggtctcccga aaccggctct     300
   tcaacctggg caccatgcag tgcctgggca cgggctggcc gggcaccaac accaccgcct     360
   ccctgggcat gtacgagtgt gaccgcgagg cactgagtct tcgctggaac tgtcgcaccc     420
   tgggcgacca gctgtccttg ctcctggggg gccgtgtcgg caacacatcc aaggctggca 480
   gccccgagcg cggtgaccag acccgcagcg gccagtggcg catctatggc agcgatgagg 540
   acctgtgtgc gcggccctac tatgaggtct acaccatcca gggcaactcc cacgggaagc 600
   cgtgcaccat ccccttcaaa tatgacaacc agtggttcca tggctgcacc agcactggcc 660
   gcgaggatgg gcacctgtgg tgcgccacca cccaggacta cggcaaggac gagcgctggg 720
   gcttctgccc catcaagggt aacgactgcg agaccttctg ggacaaggac cagctgaccg 780
   acagctgcta tcagttcaac ttccagtcca cgctgtcctg gagggaggcc tgggccagct 840
   gcgagcagca gggggcagac ctgctgagca tcacggagat ccacgagcag acctacatca 900
   atgggctcct cactggctac agctccacac tgtggattgg ccttaacgac ctggacacca 960
   gtggaggctg gcagtggtca gacaactcgc ctctcaagta cctcaactgg gagagtgatc 1020
   agccggacaa cccgagcgag gagaactgcg gggtgatccg cacggagtcg tccggcggct 1080
   ggcagaaccg cgactgcagc atcgcgctgc cctacgtctg caagaagaag cccaacgcca 1140
   cggcctccga gcccccacct cccgatgtgt gggccaacgt gaaggtggag tgtgagccca 1200
   gctggcagcc cttccagggc cactgctacc gcctgcaggc tgagaagcgc agctggcagg 1260
   agtccaagaa gatgtgtctg cggggtgggg gcgacttgct cagcatccac agcatggcgg 1320
   agctggagtt catcaccaag cagatcaagc aggaggtgga ggagctgtgg attggtctca 1380
   acgacctgaa actgcagatg aattttgagt ggtccgacgg gagcctcgtg agcttcaccc 1440
   attggcaccc ctttgagccc aacaacttcc gagacagcct ggaggactgt gtcaccatct 1500
   gggggccgga aggtcgctgg aacgacagtc cctgtaacca gtccctgccg tccatttgca 1560
   agaaggcagg ccagctgagc cagggggctg ccgaggagga ccacggctgc cggaagggtt 1620
   ggacgtggca cagcccctcc tgctactggc tgggagagga ccaggtgacc tacagtgagg 1680
   cccggcgcct gtgcaccgac cacggctctc agctggtcac catcaccaac aggttcgagc 1740
   aggcctttgt cagcagcctc atctacaact gggatggcga gtacttctgg acggccctgc 1800
   aggacctcaa cttcaccggt tccttccgct ggctcagcgg ggacgaggtc atgtacaccc 1860
   actggaaccg ggaccagcct gggtacagcc gtgggggctg cgtggccctg gccacaggca 1920
   gtgccatggg gctgtgggag gtgaagaact gcacatcgtt ccgggctcgc tacatctgcc 1980
   gccagagcct gggcacgccc gtgacgcctg agctgcctgg gctagacccc acgcccagcc 2040
   tcaccggcgc ctgtccgcag ggctggggct cagatcccaa actccggcac tgctataagg 2100
   tgttcagctc ggagcggctg caggataaga agacctgggt ccaggcccag ggggcctgcc 2160
   aggagctggg ggcccagctg ctgagcctgg ccagctacga agaggagcac tttgtggcca 2220
   acatgctcaa caagatcttc ggtgaatcag agcccgagat ccacgagcag cactggttct 2280
   ggatcggcct gaaccgccga gaccccggag ccgggcagag ctggcgctgg agtgatggac 2340
   ttgggttctc ttaccacaat ttcgaccgga gccggcacga cgatgatgac atccgcagct 2400
   gcacggtgct cgacttggcc tccctgcagt gggtggctat gcagtgcgag acgcagctgg 2460
   actggatctg caagatccct cgaggcacgg acgtgcggga gcctgacgtc agcccgcaag 2520
   gccggcggga atggctgcgt ttccaggagg ctgagtataa gttcttcgag caccactcca 2580
   cgtgggcgca ggcgcagcgc atctgcacgt ggttccaggc tgaactggtc tcagtgcaca 2640
   gccaggccga gctggacttc ctggggcaca acctgcagaa gttctctcgg ggccaggagc 2700
   agcactggtg gatcggcctg cacacctcag agagcgacgg gcgcttcagg tggacagatg 2760
   gttccattat aaacttcatc tcctgggccc ccggcaaacc tcggcccatc ggcaaggaca 2820
   agaagtgcgt gtacatgatg gccagccgag aggactgggg ggaccagagg tgcacaacag 2880
   ccttgcctta catctgcaag cggcgcaaca gcaccagaga gcagcagccc ccagacctgc 2940
   cgcccacggg gctgcccctc tggctggagc cagttcctga acaagtgttt cagaatccaa 3000
   ggccaggacc cccaggaccg ggtgaagtgg tcagaggcac agttctcctg tgaacagcaa 3060
   gaggcccaac tggtcaccat tgcaaacccc ttagagcaag cgttcatcac agccagcctg 3120
   cccaatgtga cctttgacct ttggattggc ctccatgcct cgaagaggga cttccagtgg 3180
   gtggagcagg agcctctgca gtacgccaac tgggcccctg gggagccctc tggccctagc 3240
   cctgctccca gcggcaacat accgaccagc tgtgccgtgg tcctgcatag cccctcaacc 3300
   cacttcactg gccgctggga tgaccggagc tgtgcagagg agacccatgg cttcatctgc 3360
   cagaaggcca cggacccctc cctgagccca tccccagcag cgtcgccccc tgccccgggc 3420
   gctgagctct cctacctcaa cggcaccttc cggctgctgc agaagccgct gcgctggcac 3480
   gacgccctgt tgctgtgtga gagccgaaat gccagcctgg cgcacgtgct tgacccctac 3540
   acccaggcct tcctcactca ggccgcccga gggctgcgca cgccactctg gattgggctg 3600
   gccagtgagg agggctcccg gcggtactct tgggtctcgg aggagccgct gagctacgtg 3660
   agctggcagg acggggagcc ccagcacccc gggggctgcg cctacgtgga cacggatggt 3720
   gcctggcgca ccaccagctg tgacaccaag ttgcagggtg ctgtgtgtgg agttcacggc 3780
   gggccccctc ctccgcgacg aataagctac cacgggagct gtccccaggg gctggcggac 3840
   tcggcatgga ttcccttccg agagcactgc tactccttcc atatggagct gctgctgggc 3900
   cacaaggagg cgctgcagcg ctgccagaga gcgggcggcg cggtcctgtc catcctggat 3960
   gagatggaga acgtgtttgt ctgggagcat ctgcagagct ctgagggcca gagtcggggc 4020
   gcctggctgg gcatgaactt caaccccaaa gggggcaccc tggtctggca ggacaacaca 4080
   gctgtgaact actccaactg ggggcccccg ggcctgggcc ccagcatgct gagccacaac 4140
   agctgctact ggatccagag cagcagcggg ctgtggcgcc cgggggcctg caccaacatc 4200
   agcatgggcg tcgtctgtaa gctccctcga gctgaggcga gcagcttctc cccatcagcc 4260
   ctcccggaga accccgcggc cctggtggtg gtgctgatgg cggtgctcct gctcctggct 4320
   ctgctgacgg ctgccctgat tctctaccgg cggcgacaga gcgtcgagcg tggggccttc 4380
   gagggtgccc gctacagccg cagctcctcc ggccccagcg aggccaccga gaagaacatc 4440
   ctggtgtccg acatggaaat gaacgagcag caggagtaga gccgagggcg tgggcggggc 4500
   cgagggcgtg ggcggggccg ggggagctgg gcgccccacc agctgcggtc cagtcggcct 4560
   atggggggag gtggcgccct ggggagcgct gtggggagct gaggctggca gggcctgggc 4620
   tggtggggtc cttccctccc atgagggctg ggctgaggcc tggctgagtg cagcatggcg 4680
   tttccttttg ggggggccct aaggtcttgt cacccgggcc tgtaccccca tggtcaccgg 4740
   aggcaggaca ggagcctctt tctccctgcc cccacccccc aggcctgtcc cccaaccctg 4800
   ggaccccctc ttctcgctgc aaagtctgag gagcctccct gagccctcag ccggcctctg 4860
   tcgcttctcc cctaccctgg cagctcagct cctcgtccca catcctccct gccccccacc 4920
   cgctgtccgc ccgccctgca tcctgcctgc tctcccagcc cttccttctg agccagggcc 4980
   ctggggtctg gggagccctc tcttgctgtt ggtgggggtc acagggtgag catttgtcaa 5040
   ttgtgtgcct gctggggtgg ccgtagggca tgacgggagg gttctgggct gggactgagg 5100
   accggagcat cactgtggta tcagctgggc tggagacagg tccggggaga gacaccctga 5160
   cctcctgggg atgaccgggc tgggctgggg tgtcatctcc tgccggtcag aggagggctc 5220
   tgtccctgta aagtcccctg tggggaccaa agtaagttcc ctaaggtttt caggtcctgg 5280
   ctgtagtttg gagagagaga aggtggttgg ccagggtcag tgggagctgg ggtgcgccct 5340
   ctaagggggg cccagaggag cagcacctga accccctgga gtggcccctg tgtgggggag 5400
   acgacccacc cagaaccttt ccaagaggcc ggagccccag ttagaatcac cagtcctggc 5460
   gccatggccc agggcaatgt gagcacagac tccaagacac ggtccttttt gtagttctta 5520
   atttttttta atgtgccatt attgttttaa aaagaaaaag ataaaagaaa agcaaacaaa 5580
   taaaacacct ttcagaggct tgagagagaa gggtccccac tttgttttgt tgggagtctt 5640
   gtccttcata ctgcttcaaa gatacatgtt attttggggg gcttgcatct gttttgcttc 5700
   tgtcttgggg ccacgggacc ccagccttct ctctgctcct ggggatacgt gtgagagtaa 5760
   gcattcagga gccaaaggac agtggcgtga gcttgggcaa cttggctccc ctttcaaagt 5820
   gtaaaacagg agaacaggat gcacctcccg agctccttta aggaggttga ggattaagtg 5880
   agatcacctg ggcaaagcaa tctgcacagg gccttggcgc tcgagctgta attgtcccat 5940
   aaacgtcagc tggtactgct tgtctctggg ctgtggctcc caggccagcg tgtgaggtac 6000
   ccctgccaga ccgcggtggg ggccccacac caccatgctt ccaagacaga cctgcatttg 6060
   agctgcaaga cagtgagtac acatttgtcc ctgggtagtg tgggctctga aactgccctg 6120
   aggcagggga gggggtcaca ggaccccgcc caccaccatc acctccattc 6170
<210> 5
   <211> 1478
   <212> PRT
   <213> Bos sp.
<220>
   <221> polypeptide
   <222> (1)..(1478)
   <223> wild-type Endo180 protein
<400> 5
   Met Gly Pro Gly Arg Pro Ala Leu Ala Pro Trp Pro His His Leu Leu
   1 5 10 15
   Arg Cys Ala Leu Leu Phe Gly Gly Leu His Phe Gly Arg Pro Ala Ala
   20 25 30
   Ala Ala Ala Ala Ala Leu Pro Glu Pro Asn Val Phe Val Ile Phe Ser
   35 40 45
   His Gly Leu Gln Gly Cys Leu Glu Ala Gln Gly Gly Gln Val Arg Val
   50 55 60
   Ser Pro Ala Cys Asn Ala Ser Leu Pro Ala Gln Arg Trp Lys Trp Val
   65 70 75 80
   Ser Arg Asn Arg Leu Phe Asn Leu Gly Thr Met Gln Cys Leu Gly Thr
   85 90 95
   Gly Trp Pro Gly Thr Asn Thr Thr Ala Ser Leu Gly Met Tyr Glu Cys
   100 105 110
   Asp Arg Glu Ala Leu Ser Leu Arg Trp Asn Cys Arg Thr Leu Gly Asp
   115 120 125
   Gln Leu Ser Leu Leu Leu Gly Gly Arg Val Gly Asn Thr Ser Lys Ala
   130 135 140
   Gly Ser Pro Glu Arg Gly Asp Gln Thr Arg Ser Gly Gln Trp Arg Ile
   145 150 155 160
   Tyr Gly Ser Asp Glu Asp Leu Cys Ala Arg Pro Tyr Tyr Glu Val Tyr
   165 170 175
   Thr Ile Gln Gly Asn Ser His Gly Lys Pro Cys Thr Ile Pro Phe Lys
   180 185 190
   Tyr Asp Asn Gln Trp Phe His Gly Cys Thr Ser Thr Gly Arg Glu Asp
   195 200 205
   Gly His Leu Trp Cys Ala Thr Thr Gln Asp Tyr Gly Lys Asp Glu Arg
   210 215 220
   Trp Gly Phe Cys Pro Ile Lys Gly Asn Asp Cys Glu Thr Phe Trp Asp
   225 230 235 240
   Lys Asp Gln Leu Thr Asp Ser Cys Tyr Gln Phe Asn Phe Gln Ser Thr
   245 250 255
   Leu Ser Trp Arg Glu Ala Trp Ala Ser Cys Glu Gln Gln Gly Ala Asp
   260 265 270
   Leu Leu Ser Ile Thr Glu Ile His Glu Gln Thr Tyr Ile Asn Gly Leu
   275 280 285
   Leu Thr Gly Tyr Ser Ser Thr Leu Trp Ile Gly Leu Asn Asp Leu Asp
   290 295 300
   Thr Ser Gly Gly Trp Gln Trp Ser Asp Asn Ser Pro Leu Lys Tyr Leu
   305 310 315 320
   Asn Trp Glu Ser Asp Gln Pro Asp Asn Pro Ser Glu Glu Asn Cys Gly
   325 330 335
   Val Ile Arg Thr Glu Ser Ser Gly Gly Trp Gln Asn Arg Asp Cys Ser
   340 345 350
   Ile Ala Leu Pro Tyr Val Cys Lys Lys Lys Pro Asn Ala Thr Ala Ser
   355 360 365
   Glu Pro Pro Pro Pro Asp Val Trp Ala Asn Val Lys Val Glu Cys Glu
   370 375 380
   Pro Ser Trp Gln Pro Phe Gln Gly His Cys Tyr Arg Leu Gln Ala Glu
   385 390 395 400
   Lys Arg Ser Trp Gln Glu Ser Lys Lys Met Cys Leu Arg Gly Gly Gly
   405 410 415
   Asp Leu Leu Ser Ile His Ser Met Ala Glu Leu Glu Phe Ile Thr Lys
   420 425 430
   Gln Ile Lys Gln Glu Val Glu Glu Leu Trp Ile Gly Leu Asn Asp Leu
   435 440 445
   Lys Leu Gln Met Asn Phe Glu Trp Ser Asp Gly Ser Leu Val Ser Phe
   450 455 460
   Thr His Trp His Pro Phe Glu Pro Asn Asn Phe Arg Asp Ser Leu Glu
   465 470 475 480
   Asp Cys Val Thr Ile Trp Gly Pro Glu Gly Arg Trp Asn Asp Ser Pro
   485 490 495
   Cys Asn Gln Ser Leu Pro Ser Ile Cys Lys Lys Ala Gly Gln Leu Ser
   500 505 510
   Gln Gly Ala Ala Glu Glu Asp His Gly Cys Arg Lys Gly Trp Thr Trp
   515 520 525
   His Ser Pro Ser Cys Tyr Trp Leu Gly Glu Asp Gln Val Thr Tyr Ser
   530 535 540
   Glu Ala Arg Arg Leu Cys Thr Asp His Gly Ser Gln Leu Val Thr Ile
   545 550 555 560
   Thr Asn Arg Phe Glu Gln Ala Phe Val Ser Ser Leu Ile Tyr Asn Trp
   565 570 575
   Asp Gly Glu Tyr Phe Trp Thr Ala Leu Gln Asp Leu Asn Phe Thr Gly
   580 585 590
   Ser Phe Arg Trp Leu Ser Gly Asp Glu Val Met Tyr Thr His Trp Asn
   595 600 605
   Arg Asp Gln Pro Gly Tyr Ser Arg Gly Gly Cys Val Ala Leu Ala Thr
   610 615 620
   Gly Ser Ala Met Gly Leu Trp Glu Val Lys Asn Cys Thr Ser Phe Arg
   625 630 635 640
   Ala Arg Tyr Ile Cys Arg Gln Ser Leu Gly Thr Pro Val Thr Pro Glu
   645 650 655
   Leu Pro Gly Leu Asp Pro Thr Pro Ser Leu Thr Gly Ala Cys Pro Gln
   660 665 670
   Gly Trp Gly Ser Asp Pro Lys Leu Arg His Cys Tyr Lys Val Phe Ser
   675 680 685
   Ser Glu Arg Leu Gln Asp Lys Lys Thr Trp Val Gln Ala Gln Gly Ala
   690 695 700
   Cys Gln Glu Leu Gly Ala Gln Leu Leu Ser Leu Ala Ser Tyr Glu Glu
   705 710 715 720
   Glu His Phe Val Ala Asn Met Leu Asn Lys Ile Phe Gly Glu Ser Glu
   725 730 735
   Pro Glu Ile His Glu Gln His Trp Phe Trp Ile Gly Leu Asn Arg Arg
   740 745 750
   Asp Pro Gly Ala Gly Gln Ser Trp Arg Trp Ser Asp Gly Leu Gly Phe
   755 760 765
   Ser Tyr His Asn Phe Asp Arg Ser Arg His Asp Asp Asp Asp Ile Arg
   770 775 780
   Ser Cys Thr Val Leu Asp Leu Ala Ser Leu Gln Trp Val Ala Met Gln
   785 790 795 800
   Cys Glu Thr Gln Leu Asp Trp Ile Cys Lys Ile Pro Arg Gly Thr Asp
   805 810 815
   Val Arg Glu Pro Asp Val Ser Pro Gln Gly Arg Arg Glu Trp Leu Arg
   820 825 830
   Phe Gln Glu Ala Glu Tyr Lys Phe Phe Glu His His Ser Thr Trp Ala
   835 840 845
   Gln Ala Gln Arg Ile Cys Thr Trp Phe Gln Ala Glu Leu Val Ser Val
   850 855 860
   His Ser Gln Ala Glu Leu Asp Phe Leu Gly His Asn Leu Gln Lys Phe
   865 870 875 880
   Ser Arg Gly Gln Glu Gln His Trp Trp Ile Gly Leu His Thr Ser Glu
   885 890 895
   Ser Asp Gly Arg Phe Arg Trp Thr Asp Gly Ser Ile Ile Asn Phe Ile
   900 905 910
   Ser Trp Ala Pro Gly Lys Pro Arg Pro Ile Gly Lys Asp Lys Lys Cys
   915 920 925
   Val Tyr Met Met Ala Ser Arg Glu Asp Trp Gly Asp Gln Arg Cys Thr
   930 935 940
   Thr Ala Leu Pro Tyr Ile Cys Lys Arg Arg Asn Ser Thr Arg Glu Gln
   945 950 955 960
   Gln Pro Pro Asp Leu Pro Pro Thr Gly Gly Cys Pro Ser Gly Trp Ser
   965 970 975
   Gln Phe Leu Asn Lys Cys Phe Arg Ile Gln Gly Gln Asp Pro Gln Asp
   980 985 990
   Arg Val Lys Trp Ser Glu Ala Gln Phe Ser Cys Glu Gln Gln Glu Ala
   995 1000 1005
   Gln Leu Val Thr Ile Ala Asn Pro Leu Glu Gln Ala Phe Ile Thr
   1010 1015 1020
   Ala Ser Leu Pro Asn Val Thr Phe Asp Leu Trp Ile Gly Leu His
   1025 1030 1035
   Ala Ser Lys Arg Asp Phe Gln Trp Val Glu Gln Glu Pro Leu Gln
   1040 1045 1050
   Tyr Ala Asn Trp Ala Pro Gly Glu Pro Ser Gly Pro Ser Pro Ala
   1055 1060 1065
   Pro Ser Gly Asn Ile Pro Thr Ser Cys Ala Val Val Leu His Ser
   1070 1075 1080
   Pro Ser Thr His Phe Thr Gly Arg Trp Asp Asp Arg Ser Cys Ala
   1085 1090 1095
   Glu Glu Thr His Gly Phe Ile Cys Gln Lys Ala Thr Asp Pro Ser
   1100 1105 1110
   Leu Ser Pro Ser Pro Ala Ala Ser Pro Pro Ala Pro Gly Ala Glu
   1115 1120 1125
   Leu Ser Tyr Leu Asn Gly Thr Phe Arg Leu Leu Gln Lys Pro Leu
   1130 1135 1140
   Arg Trp His Asp Ala Leu Leu Leu Cys Glu Ser Arg Asn Ala Ser
   1145 1150 1155
   Leu Ala His Val Leu Asp Pro Tyr Thr Gln Ala Phe Leu Thr Gln
   1160 1165 1170
   Ala Ala Arg Gly Leu Arg Thr Pro Leu Trp Ile Gly Leu Ala Ser
   1175 1180 1185
   Glu Glu Gly Ser Arg Arg Tyr Ser Trp Val Ser Glu Glu Pro Leu
   1190 1195 1200
   Ser Tyr Val Ser Trp Gln Asp Gly Glu Pro Gln His Pro Gly Gly
   1205 1210 1215
   Cys Ala Tyr Val Asp Thr Asp Gly Ala Trp Arg Thr Thr Ser Cys
   1220 1225 1230
   Asp Thr Lys Leu Gln Gly Ala Val Cys Gly Val His Gly Gly Pro
   1235 1240 1245
   Pro Pro Pro Arg Arg Ile Ser Tyr His Gly Ser Cys Pro Gln Gly
   1250 1255 1260
   Leu Ala Asp Ser Ala Trp Ile Pro Phe Arg Glu His Cys Tyr Ser
   1265 1270 1275
   Phe His Met Glu Leu Leu Leu Gly His Lys Glu Ala Leu Gln Arg
   1280 1285 1290
   Cys Gln Arg Ala Gly Gly Ala Val Leu Ser Ile Leu Asp Glu Met
   1295 1300 1305
   Glu Asn Val Phe Val Trp Glu His Leu Gln Ser Ser Glu Gly Gln
   1310 1315 1320
   Ser Arg Gly Ala Trp Leu Gly Met Asn Phe Asn Pro Lys Gly Gly
   1325 1330 1335
   Thr Leu Val Trp Gln Asp Asn Thr Ala Val Asn Tyr Ser Asn Trp
   1340 1345 1350
   Gly Pro Pro Gly Leu Gly Pro Ser Met Leu Ser His Asn Ser Cys
   1355 1360 1365
   Tyr Trp Ile Gln Ser Ser Ser Gly Leu Trp Arg Pro Gly Ala Cys
   1370 1375 1380
   Thr Asn Ile Ser Met Gly Val Val Cys Lys Leu Pro Arg Ala Glu
   1385 1390 1395
   Ala Ser Ser Phe Ser Pro Ser Ala Leu Pro Glu Asn Pro Ala Ala
   1400 1405 1410
   Leu Val Val Val Leu Met Ala Val Leu Leu Leu Leu Ala Leu Leu
   1415 1420 1425
   Thr Ala Ala Leu Ile Leu Tyr Arg Arg Arg Gln Ser Val Glu Arg
   1430 1435 1440
   Gly Ala Phe Glu Gly Ala Arg Tyr Ser Arg Ser Ser Ser Gly Pro
   1445 1450 1455
   Ser Glu Ala Thr Glu Lys Asn Ile Leu Val Ser Asp Met Glu Met
   1460 1465 1470
   Asn Glu Gln Gln Glu
   1475
<210> 6
   <211> 1002
   <212> PRT
   <213> Bos sp.
<220>
   <221> polypeptide
   <222> (1)..(1002)
   <223> truncated Endo180 protein
<400> 6
   Met Gly Pro Gly Arg Pro Ala Leu Ala Pro Trp Pro His His Leu Leu
   1 5 10 15
   Arg Cys Ala Leu Leu Phe Gly Gly Leu His Phe Gly Arg Pro Ala Ala
   20 25 30
   Ala Ala Ala Ala Ala Leu Pro Glu Pro Asn Val Phe Val Ile Phe Ser
   35 40 45
   His Gly Leu Gln Gly Cys Leu Glu Ala Gln Gly Gly Gln Val Arg Val
   50 55 60
   Ser Pro Ala Cys Asn Ala Ser Leu Pro Ala Gln Arg Trp Lys Trp Val
   65 70 75 80
   Ser Arg Asn Arg Leu Phe Asn Leu Gly Thr Met Gln Cys Leu Gly Thr
   85 90 95
   Gly Trp Pro Gly Thr Asn Thr Thr Ala Ser Leu Gly Met Tyr Glu Cys
   100 105 110
   Asp Arg Glu Ala Leu Ser Leu Arg Trp Asn Cys Arg Thr Leu Gly Asp
   115 120 125
   Gln Leu Ser Leu Leu Leu Gly Gly Arg Val Gly Asn Thr Ser Lys Ala
   130 135 140
   Gly Ser Pro Glu Arg Gly Asp Gln Thr Arg Ser Gly Gln Trp Arg Ile
   145 150 155 160
   Tyr Gly Ser Asp Glu Asp Leu Cys Ala Arg Pro Tyr Tyr Glu Val Tyr
   165 170 175
   Thr Ile Gln Gly Asn Ser His Gly Lys Pro Cys Thr Ile Pro Phe Lys
   180 185 190
   Tyr Asp Asn Gln Trp Phe His Gly Cys Thr Ser Thr Gly Arg Glu Asp
   195 200 205
   Gly His Leu Trp Cys Ala Thr Thr Gln Asp Tyr Gly Lys Asp Glu Arg
   210 215 220
   Trp Gly Phe Cys Pro Ile Lys Gly Asn Asp Cys Glu Thr Phe Trp Asp
   225 230 235 240
   Lys Asp Gln Leu Thr Asp Ser Cys Tyr Gln Phe Asn Phe Gln Ser Thr
   245 250 255
   Leu Ser Trp Arg Glu Ala Trp Ala Ser Cys Glu Gln Gln Gly Ala Asp
   260 265 270
   Leu Leu Ser Ile Thr Glu Ile His Glu Gln Thr Tyr Ile Asn Gly Leu
   275 280 285
   Leu Thr Gly Tyr Ser Ser Thr Leu Trp Ile Gly Leu Asn Asp Leu Asp
   290 295 300
   Thr Ser Gly Gly Trp Gln Trp Ser Asp Asn Ser Pro Leu Lys Tyr Leu
   305 310 315 320
   Asn Trp Glu Ser Asp Gln Pro Asp Asn Pro Ser Glu Glu Asn Cys Gly
   325 330 335
   Val Ile Arg Thr Glu Ser Ser Gly Gly Trp Gln Asn Arg Asp Cys Ser
   340 345 350
   Ile Ala Leu Pro Tyr Val Cys Lys Lys Lys Pro Asn Ala Thr Ala Ser
   355 360 365
   Glu Pro Pro Pro Pro Asp Val Trp Ala Asn Val Lys Val Glu Cys Glu
   370 375 380
   Pro Ser Trp Gln Pro Phe Gln Gly His Cys Tyr Arg Leu Gln Ala Glu
   385 390 395 400
   Lys Arg Ser Trp Gln Glu Ser Lys Lys Met Cys Leu Arg Gly Gly Gly
   405 410 415
   Asp Leu Leu Ser Ile His Ser Met Ala Glu Leu Glu Phe Ile Thr Lys
   420 425 430
   Gln Ile Lys Gln Glu Val Glu Glu Leu Trp Ile Gly Leu Asn Asp Leu
   435 440 445
   Lys Leu Gln Met Asn Phe Glu Trp Ser Asp Gly Ser Leu Val Ser Phe
   450 455 460
   Thr His Trp His Pro Phe Glu Pro Asn Asn Phe Arg Asp Ser Leu Glu
   465 470 475 480
   Asp Cys Val Thr Ile Trp Gly Pro Glu Gly Arg Trp Asn Asp Ser Pro
   485 490 495
   Cys Asn Gln Ser Leu Pro Ser Ile Cys Lys Lys Ala Gly Gln Leu Ser
   500 505 510
   Gln Gly Ala Ala Glu Glu Asp His Gly Cys Arg Lys Gly Trp Thr Trp
   515 520 525
   His Ser Pro Ser Cys Tyr Trp Leu Gly Glu Asp Gln Val Thr Tyr Ser
   530 535 540
   Glu Ala Arg Arg Leu Cys Thr Asp His Gly Ser Gln Leu Val Thr Ile
   545 550 555 560
   Thr Asn Arg Phe Glu Gln Ala Phe Val Ser Ser Leu Ile Tyr Asn Trp
   565 570 575
   Asp Gly Glu Tyr Phe Trp Thr Ala Leu Gln Asp Leu Asn Phe Thr Gly
   580 585 590
   Ser Phe Arg Trp Leu Ser Gly Asp Glu Val Met Tyr Thr His Trp Asn
   595 600 605
   Arg Asp Gln Pro Gly Tyr Ser Arg Gly Gly Cys Val Ala Leu Ala Thr
   610 615 620
   Gly Ser Ala Met Gly Leu Trp Glu Val Lys Asn Cys Thr Ser Phe Arg
   625 630 635 640
   Ala Arg Tyr Ile Cys Arg Gln Ser Leu Gly Thr Pro Val Thr Pro Glu
   645 650 655
   Leu Pro Gly Leu Asp Pro Thr Pro Ser Leu Thr Gly Ala Cys Pro Gln
   660 665 670
   Gly Trp Gly Ser Asp Pro Lys Leu Arg His Cys Tyr Lys Val Phe Ser
   675 680 685
   Ser Glu Arg Leu Gln Asp Lys Lys Thr Trp Val Gln Ala Gln Gly Ala
   690 695 700
   Cys Gln Glu Leu Gly Ala Gln Leu Leu Ser Leu Ala Ser Tyr Glu Glu
   705 710 715 720
   Glu His Phe Val Ala Asn Met Leu Asn Lys Ile Phe Gly Glu Ser Glu
   725 730 735
   Pro Glu Ile His Glu Gln His Trp Phe Trp Ile Gly Leu Asn Arg Arg
   740 745 750
   Asp Pro Gly Ala Gly Gln Ser Trp Arg Trp Ser Asp Gly Leu Gly Phe
   755 760 765
   Ser Tyr His Asn Phe Asp Arg Ser Arg His Asp Asp Asp Asp Ile Arg
   770 775 780
   Ser Cys Thr Val Leu Asp Leu Ala Ser Leu Gln Trp Val Ala Met Gln
   785 790 795 800
   Cys Glu Thr Gln Leu Asp Trp Ile Cys Lys Ile Pro Arg Gly Thr Asp
   805 810 815
   Val Arg Glu Pro Asp Val Ser Pro Gln Gly Arg Arg Glu Trp Leu Arg
   820 825 830
   Phe Gln Glu Ala Glu Tyr Lys Phe Phe Glu His His Ser Thr Trp Ala
   835 840 845
   Gln Ala Gln Arg Ile Cys Thr Trp Phe Gln Ala Glu Leu Val Ser Val
   850 855 860
   His Ser Gln Ala Glu Leu Asp Phe Leu Gly His Asn Leu Gln Lys Phe
   865 870 875 880
   Ser Arg Gly Gln Glu Gln His Trp Trp Ile Gly Leu His Thr Ser Glu
   885 890 895
   Ser Asp Gly Arg Phe Arg Trp Thr Asp Gly Ser Ile Ile Asn Phe Ile
   900 905 910
   Ser Trp Ala Pro Gly Lys Pro Arg Pro Ile Gly Lys Asp Lys Lys Cys
   915 920 925
   Val Tyr Met Met Ala Ser Arg Glu Asp Trp Gly Asp Gln Arg Cys Thr
   930 935 940
   Thr Ala Leu Pro Tyr Ile Cys Lys Arg Arg Asn Ser Thr Arg Glu Gln
   945 950 955 960
   Gln Pro Pro Asp Leu Pro Pro Thr Gly Leu Pro Leu Trp Leu Glu Pro
   965 970 975
   Val Pro Glu Gln Val Phe Gln Asn Pro Arg Pro Gly Pro Pro Gly Pro
   980 985 990
   Gly Glu Val Val Arg Gly Thr Val Leu Leu
   995 1000
<210> 7
   <211> 14
   <212> DNA
   <213> Artificial
<220>
   <223> primer
<400> 7
   ctgccgccca cggg 14
<210> 8
   <211> 14
   <212> DNA
   <213> Artificial
<220>
   <223> primer
<400> 8
   ctgccgccca cagg 14
<210> 9
   <211> 18
   <212> DNA
   <213> Artificial
<220>
   <223> primer
<400> 9
   gcgcaacagc accagaga 18
<210> 10
   <211> 23
   <212> DNA
   <213> Artificial
<220>
   <223> primer
<400> 10
   ctccctacct tgttcaggaa ctg 23

## Description

This invention relates to methods, assay and kit for the detection of animals that are carriers of Crooked Tail Syndrome (CTS) or "pig-like calves".

### Field of the invention

Intense selection for desired production traits in livestock often results in an increase of the rate of inbreeding. This causes the frequent emergence of inherited defects which can affect such a high proportion of the population that they become a major problem. Examples of genetic defects that have caused major problems in livestock are bovine leucocyte adhesion deficiency (BLAD), congenital vertebral malformation (CVM) and others.

Most of these disorders are characterized by a recessive mode of inheritance: animals can be homozygous for the normal allele (+/+), homozygous for the defective allele (D/D), or heterozygous (+/D). D/D animals are afflicted by the disease, while +/+ and +/D animals are generally healthy. +/D animals, however, are said to be "carriers" and will transmit the D allele to half of their offspring. The mating between two carrier animals will produce one fourth of affected D/D offspring.

If one could identify +/D animals despite the fact that they don't exhibit the symptoms of the disease, it would be possible to eliminate them from breeding programs or at least avoid mating between carriers. This would allow immediate eradication of the genetic defect from the population and avoid the economic losses associated with it.

It has recently become possible to identify the genes underlying genetic defects and the mutations in them which cause an allele to be D(efective). It has also recently become possible to use molecular biology techniques to rapidly analyze a DNA sample from an individual and to determine whether it contains a D allele, i.e. it is now possible to detect carriers if the gene and causal mutation underlying the defect are known.

### Background of the invention

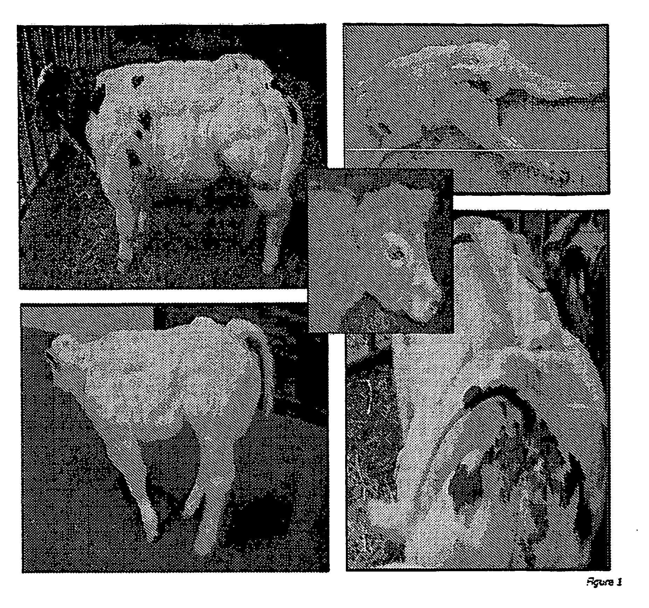

End of the year 2006 field veterinarians become very concerned by an emerging defect in Belgian Blue cattle, a breed well known for its extreme muscularity. In many farms, 3-4% of calves were affected by a inherited disease causing Crooked Tail Syndrome (CTS). The main clinical signs are extreme muscularity, growth retardation, postural abnormality, shortened head and crooked tail. In addition, a few cases exhibit scoliosis, joint rigidity, and spastic paresis of the hind limbs (Figure 1). Even if the defect was not lethal, euthanasia is sometimes necessary and economic losses due to growth retardation and clinical complications is of importance.

The object of the present invention was to provide a diagnostic method, assay and kit for detecting whether or not a bovine individual is carrier of Crooked Tail Syndrome or is suffering from or will develop said disease.

### Summary of the invention

In order to solve the object the present invention provides the use of the *MRC2* gene locus coding for the Endo180 protein as marker for detecting whether or not a bovine individual is carrier of Crooked Tail Syndrome (CTS).

The present invention further provides an in-vitro method for detecting whether or not a bovine individual is carrier of Crooked Tail Syndrome (CTS), by analyzing the *MRC2* gene locus coding for the Endo180 protein.

In addition the present invention provides an assay for detecting whether or not a bovine individual is carrier of Crooked Tail Syndrome (CTS), wherein the *MRC2* gene locus coding for the Endo180 protein is analyzed.

In particular, the present invention revealed that a mutated allele of *MRC2* gene is responsible for Crooked tail, growth retardation and muscular hypertrophy syndrome (CTS) in bovine individuals.

In a preferred embodiment the analysis of the bovine *MRC2* gene locus includes amplifying the DNA in the presence of primers hybridizing either with a nucleic acid molecule having the sequence identified as SEQ ID N°1 or 3 or with a nucleic acid molecule having the sequence identified as SEQ ID N°2 or 4.

In a preferred embodiment of the present invention a bovine individual having a mutated *MRC2* gene is judged to have carrier status for Crooked Tail Syndrome (CTS). A mutated *MRC2* gene represents the disease-causing allele which is responsible for Crooked Tail Syndrome (CTS) in cattle. This mutation may be of any type as long as the mutation of said *MRC2* gene is rendering inactive or removing any one or more of the transmembrane domain, the cytoplasmic domain or one or more of the C-type lectin domains of the Endo180 protein. In particular, the disease-causing mutation of the *MRC2* gene removes at least three out of eight C-type lectin domains, the transmembrane domain and the cytoplasmic domain of the Endo180 protein.

Preferably, a bovine individual having a mutated *MRC2* gene coding for a truncated Endo180 protein is judged to have carrier status for Crooked Tail Syndrome (CTS). Further preferred, a bovine individual having an allele of the *MRC2* gene which is truncated in that way that any one or more of the transmembrane domain, the cytoplasmic domain or one or more of the C-type lectin domains of the Endo180 protein is rendered inactive or removed, is judged to have carrier status for Crooked Tail Syndrome (CTS). In particular, the the disease-causing truncation of the *MRC2* gene removes at least three out of eight C-type lectin domains, the transmembrane domain and the cytoplasmic domain of the Endo180 protein.

In a further preferred embodiment of the present invention the presence of an allele of the *MRC2* sequence, which is characterized by a two base pair deletion at nucleotide positions 2904 and 2905 of the coding sequence resulting in a frame-shift of the reading frame and resulting in a truncation of the Endo180 protein, indicates carrier status.

Further preferred, the presence of an allele of the *MRC2* sequence, which is characterized by the nucleotides AG at nucleotide positions 2904 and 2905 of the coding sequence and corresponds to the wild-type Endo180 protein, indicates non-carrier status.

In a further preferred embodiment the genomic DNA is analyzed and the presence of the sequence SEQ ID NO: 2 indicates carrier status. Whereas the presence of the sequence SEQ ID NO: 1 indicates non-carrier status.

Further preferred, the RNA is transcribed into cDNA by reverse transcription and said cDNA is analyzed and the presence of the sequence SEQ ID NO: 4 indicates carrier status. Whereas the presence of the sequence SEQ ID NO: 3 indicates non-carrier status.

In a particularly preferred embodiment of the present invention the analysis of the bovine *MRC2* gene locus includes the use of a nucleic acid probe hybridizing with a nucleic acid molecule having the sequence identified as SEQ ID N°1 or 3 for determining that the *MRC2* sequence is characterized by AG residues at nucleotide positions 2904 and 2905 of the coding sequence, corresponding to a wild-type Endo180 protein. In a further preferred embodiment of the present invention the analysis of the bovine *MRC2* gene locus or includes the use of a nucleic acid probe hybridizing with a nucleic acid molecule having the sequence identified as SEQ ID N°2 or 4 for determining that the *MRC2* sequence is characterized by a two bp deletion at nucleotide positions 2904 and 2905 of the coding sequence corresponding to truncated Endo180 protein.

In preferred embodiments of the use, method and assay primers are used which allow the amplification of a sequence selected from SEQ ID NO: 1, SEQ ID NO: 2, SEQ ID NO: 3 and SEQ ID NO: 4 or a partial sequence thereof, wherein the partial sequence comprises at least the nucleotide positions 2904 and 2905 of the coding sequence. Further preferred, the use, method and assay make use of forward and reverse primers SEQ ID NO:9 and ID NO:10, respectively. Said primers are used for the amplification of a sequence selected from SEQ ID NO: 1, SEQ ID NO: 2, SEQ ID NO: 3 and SEQ ID NO: 4 or a partial sequence thereof as mentioned above, before the step of analyzing if or if not the disease-causing allele of MRC2 is present.

In another preferred embodiment, the present invention makes use of a primer comprising the sequence 5'-CTG CCG CCC AC 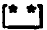 GGG-3' (SEQ ID NO: 7) or its complementary sequence for detecting the disease-causing allele of the *MRC2* gene and thus indicating the carrier status. In particularly preferred embodiment, the present invention makes use of a primer having the sequence 5'-CTG CCG CCC AC 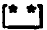 GGG-3' (SEQ ID NO: 7) or its complementary sequence for detecting the disease-causing allele of the *MRC2* gene and thus indicating the carrier status

Further preferred, in the present invention a primer comprising the sequence 5'-CTG CCG CCC AC**A G**G-3' (SEQ ID NO: 8) or its complementary sequence is used for detecting the wild-type allele of the *MRC2* gene and thus indicating the non-carrier status. In particularly preferred embodiment, in the present invention a primer having the sequence 5'-CTG CCG CCC AC**A G**G-3' (SEQ ID NO: 8) or its complementary sequence is used for detecting the wild-type allele of the *MRC2* gene and thus indicating the non-carrier status.

In a still further preferred embodiment, a bovine individual which is revealed by the use, method and assay according to the present invention as being homozygous in respect to the disease-causing allele in the *MRC2* gene locus is judged to have or to develop Crooked Tail Syndrome (CTS).

The use, the method or the assay according to the present invention for detecting whether or not a bovine individual is carrier of Crooked Tail Syndrome involves the isolation of a sample from the bovine individual to be tested, wherein the sample may be any material containing nucleated cells from said animal including blood, sperm, hair, milk, tissues. The tissues may be selected from chondrocytes, muscle, skin and others.

The present invention also provides a reagent kit for detecting whether or not a bovine individual is carrier of Crooked Tail Syndrome (CTS), comprising
a) a primer comprising the sequence 5'-CTG CCG CCC AC 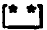 GGG-3' (SEQ ID NO: 7) or its complementary sequence for detecting the disease-causing allele of the *MRC2* gene and thus indicating the carrier status; and optionally
b) a primer comprising the sequence 5'-CTG CCG CCC AC**A G**G-3' (SEQ ID NO: 8) or its complementary sequence for detecting the wild-type allele of the *MRC2* gene and thus indicating the non-carrier status.

The highlighted "**AG**" nucleotides in the sequence SEQ ID NO: 8 (5'-CTG CCG CCC AC**A G**G-3') indicate the two base pairs of the wild-type at the nucleotide position 2904 and 2905 of the coding region of the *MRC2* gene, which in the disease-causing allele are deleted. The two asterisk in brackets in the sequence SEQ ID NO: 7 (5'-CTG CCG CCC AC 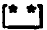 GGG-3') indicate the two base pair deletion at position 2904 and 2905 of the coding region of the mutant *MRC2* gene.

In preferred embodiments of the kit primers are included which allow the amplification of a sequence selected from SEQ ID NO: 1, SEQ ID NO: 2, SEQ ID NO: 3 and SEQ ID NO: 4 or a partial sequence thereof, wherein the partial sequence comprises at least the nucleotide positions 2904 and 2905 of the coding sequence. Further preferred, the kit comprises forward and reverse primers SEQ ID NO:9 and primer SEQ ID NO:10, respectively.

The present invention is based on the identification of a gene and its mutation, the latter causing Crooked Tail Syndrome (CTS) in cattle. As mentioned above, in 2006 field veterinarians become very concerned by an emerging defect in Belgian Blue cattle, a breed well known for its extreme muscularity. In many farms 3-4% of calves were affected. Such a high incidence leads the present inventors to perform a detailed clinical study to characterize the defect and try to determine its precise aetiology. About fifty affected calves were clinically examined, blood samples collected and their pedigrees scrutinized. Pedigree analysis revealed that CTS is in agreement with an autosomal recessive mode of inheritance of the defect. The main clinical signs were extreme muscularity, growth retardation, postural abnormality, shortened head and crooked tail. In addition, a few cases exhibited scoliosis, joint rigidity, and spastic paresis of the hind limbs (Figure 1). Expressivity of these last signs was variable. Even if the defect was not lethal, euthanasia was sometimes necessary and economic losses due to growth retardation and clinical complications was important.

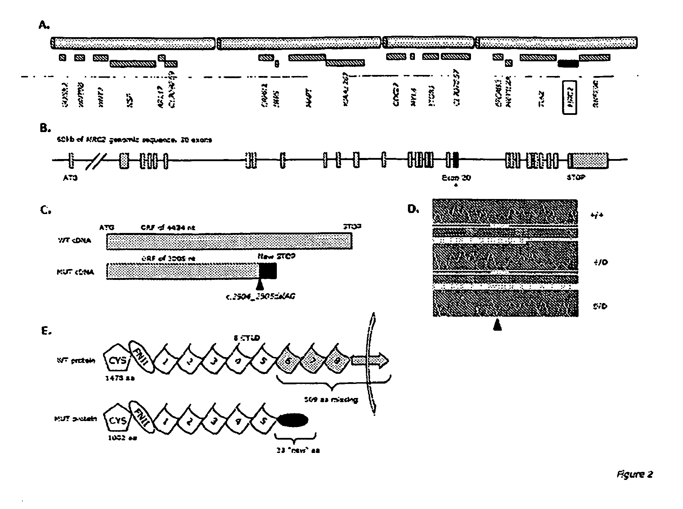

By using a technique called positional cloning (1) (Strachan and Read, 2004) the gene causing CTS could be identified. Genome scans were performed using DNA samples from affected individuals and controls using a custom made 60K Illumina SNP panel. A unique identical-by-descent (IBD) chromosomal segment of 2.42 Mb, shared by all affected individuals, was mapped on bovine chromosome 19, proving the hereditary aetiology of this new syndrome (2) (Charlier *et al.*, 2008). Candidate genes in this interval were analyzed in order to identify the causative mutation to be able to develop a diagnostic test (Figure 2A.).

During the studies for the present invention a mutation in the *MRC2* (Mannose Receptor C type 2) gene coding for the Endocytic Transmembrane Glycoprotein Endo180 was identified. The Endo180 protein is the fourth member of the mannose receptor family. The protein Endo180 functions in cells as a collagen binding and internalization receptor and plays a physiological role in mediating collagen matrix remodelling during tissue development and homeostasis (3)(Wienke *et al.*, 2003). In addition to collagens, Endo180 has been demonstrated to have two other binding activities. Firstly, Endo180 can form a trimolecular cell surface complex with prouPA and uPAR (4)(Behrendt *et al.*, 2000). Secondly, Endo180 is a functional C-type lectin showing Ca²⁺-dependent binding to mannose, fucose, and N-acetylglucosamine (5)(East *et al.*, 2002). Endo180 contains an N-terminal cysteine-rich or ricin-type domain, a fibronectin type II (FNII) domain, 8 C-type-lectin domains, a single transmembrane domain, and a short cytoplasmic domain.

The mutation identified in Belgian Blue cattle is a two base pair deletion in exon 20 of the *MRC2* gene inducing a frame-shift in the open reading frame resulting in a truncated protein, which is about 30% shorter than the wild-type Endo180 protein, missing 3 C-type lectin domains and, more importantly, the transmembrane domain as well as the cytoplasmic domain (Figure 2B., C., D., E.). In consequence, this truncated protein will be unable to uptake and internalize the collagen or to play any receptor function.

As a result of this invention, it is now possible to detect +/D carrier animals by means of simple genetic tests performed on a nucleic acid extracted from biological samples originating from said animals.

**Detailed description of the preferred embodiments**

The present invention provides a diagnostic method for detecting whether or not a bovine individual is carrier of Crooked Tail Syndrome.

The present invention also provides the use of the *MRC2* gene locus coding for the Endo180 protein as marker for detecting whether or not a bovine individual is carrier of Crooked Tail Syndrome.

The present invention further provides an in-vitro method for detecting whether or not a bovine individual is carrier of Crooked Tail Syndrome (CTS), by analyzing the *MRC2* gene locus coding for the Endo180 protein.

In one aspect, the present invention provides a method for determining whether a bovine is carrier of CTS by analyzing its genomic DNA. The method includes obtaining a sample of material containing genomic DNA from the bovine and ascertaining (i) whether a DNA sequence corresponding to the bovine *MRC2* coding for the Endo180 protein is present in the sample, and (ii) whether this *MRC2* sequence is characterized by a two bp deletion at position 2904-2905 of the cDNA (c.2904_2905delAG), leading to a truncated Endo180 protein (*L970fsX1002*).

The sample can be any material containing nucleated cells from said animal including blood, sperm, hair, milk as well as any other tissues.

There are several methods known by those skilled in the art (6) for determining whether a particular nucleotide sequence is present in a DNA sample. These include the amplification of a DNA segment encompassing the *MRC2* c.2904_2905delAG mutation by means of the polymerase chain reaction and interrogate the *MRC2* c.2904_2905delAG nucleotide positions by means of allele specific hybridization, or the 5'exonuclease assay (Taqman assay), or the use of allele-specific restriction enzymes, or direct sequencing, or the oligonucleotide ligation assay, or pyrosequencing, or the invader assay, or minisequencing, or DHPLC, or SSCP, or combinations of these methods. Alternatively the gene sequence and mutation can be ascertained by means of allele specific PCRs using primers that are specific for either the AG or the deleted allele. This list of methods is not meant to be exclusive, but just to illustrate the diversity of available methods. Some of these methods can be performed in microarray format.

In another aspect, the present invention provides a method for determining whether a bovine is carrier of CTS by analyzing its RNA. The method includes obtaining a sample of material containing RNA from the bovine, optionally converting the RNA to cDNA by reverse transcription; and ascertaining (i) whether a DNA sequence corresponding to the bovine *MRC2* coding for the Endo180 protein is present in the sample, and (ii) whether this *MRC2* sequence is characterized by an AG or a 2 pb deletion at position c.2904_2905 of the coding region, corresponding to respectively a functional wild-type Endo180 protein or a non functional truncated protein missing 509 amino acids at its C-terminal part.

The sample can be any material containing nucleated cells from said animal including blood, sperm, hair, milk as well as any other tissue, especially chondrocytes.

There are several methods known by those skilled in the art for determining whether a particular nucleotide sequence is present in a RNA sample. These include the conversion of the RNA in cDNA by means of a reverse transcriptase, and the application of the methods mentioned above or variants thereof that are known by those skilled in the art to ascertain the presence of the AG or the 2 bp deletion at position c.2904_2905.

The method thus includes the nucleic acid probes, including oligonucleotide probes, that are complementary to the "+" (*MRC2* c.2904_2905 AG) and / or "D" (*MRC2* c.2904_2905 Δ; "D" corresponds to "defect" or "delta") allele of the bovine *MRC2* gene, i.e. that have the ability to hybridize to the corresponding sequences either in genomic DNA, or in cDNA or in a PCR product derived from either of these.

### Sequence listings

The sequence listings show the sequence SEQ ID N°1 which represents the wild-type *MRC2* genomic sequence. The sequence SEQ ID N°2 represents the mutated *MRC2* genomic sequence. The sequence SEQ ID N°3 represents the wild-type *MRC2* cDNA sequence. In the given sequence the coding region starts with the nucleotide position 45. The nucleotide positions 2904 and 2905 of the coding region therefore is in position 2948 and 2949 of this sequence. The sequence SEQ ID N°4 represents the mutated *MRC2* cDNA sequence. Here again the coding region starts with the nucleotide position 45. The nucleotide positions 2904 and 2905 of the coding region which are deleted in this sequence therefore are beyond the nucleotide position 2947.

The polypeptide sequence SEQ ID N°5 represents the wild-type Endo180 protein having 1478 amino acids in length. The sequence SEQ ID N°6 represents the truncated Endo180 protein having 1002 amino acids in length. Due to the frame-shift in the mutated gene *MRC2* the truncated End0180 protein differs in its sequence from the wild-type protein in amino acid positions 970 to 1002, while amino acid positions 1003 to 1478 of the wild-type are completely missing.

The sequence SEQ ID NO: 7 is used for detecting the disease-causing allele of the *MRC2* gene and thus indicating the carrier status. Whereas the sequence SEQ ID NO: 8 is used for detecting the wild-type allele of the *MRC2* gene and thus indicating the non-carrier status. The sequences SEQ ID NO: 9 and 10 represent forward and reverse primers for the PCR amplification of the template DNA sequence.

### Figures

Figure 1 illustrates the phenotypic details of CTS. Four main clinical features were observed in more than 90% of the cases: (i) clear growth retardation, (ii) a shortening and enlargement of the head, (iii) an extreme muscular hypertrophy well visible for back muscles (ilio-spinal) with a characteristic lombo-sacral stair at the gluteus medius anchor, (iv) a tail with high implantation, deviated from the root. In addition, other symptoms were recurrent in a substantial fraction of the cases: (i) spastic paresis of the hind limbs (in 36%), often associated with straight hock (63%), (ii) a third showed abnormal fore limbs (too short, straight, in extension) and finally, (iii) a fifth was more severely affected and exhibited a pronounced scoliosis with asymmetric back muscle development.

Figure 2 shows that CTS is due to a 2bp deletion in *MRC2*. **A.** Gene content of the 2.42Mb identical-by-descent chromosomal segment; the MRC2 gene is highlighted in black. **B.** Intron-exon organization of the *MRC2* gene; mutated exon 20 is highlighted in black. **C.** Schematic representation of the wild-type and the mutated *MRC2* cDNA indicating the respective position of the deletion and the new stop codon. Due to the frame-shift the C-terminal sequence of the mutated Endo180 protein differs from the wild-type sequence (highlighted in black). **D.** Sequence traces for animals of the three *MRC2* genotypes (+/+; D/+ and D/D); the black triangle pinpoints the precise position of the 2bp deletion. **E.** Schematic representation of wild-type and truncated Endo180 proteins showing the lack of three C-type lectin domains (CTLD), the transmembrane domain and the cytoplasmic domain in the mutated form. Due to the frame-shift the C-terminal sequence of the mutated Endo180 protein contains 33 amino acids which differs from the wild-type sequence (highlighted in black).

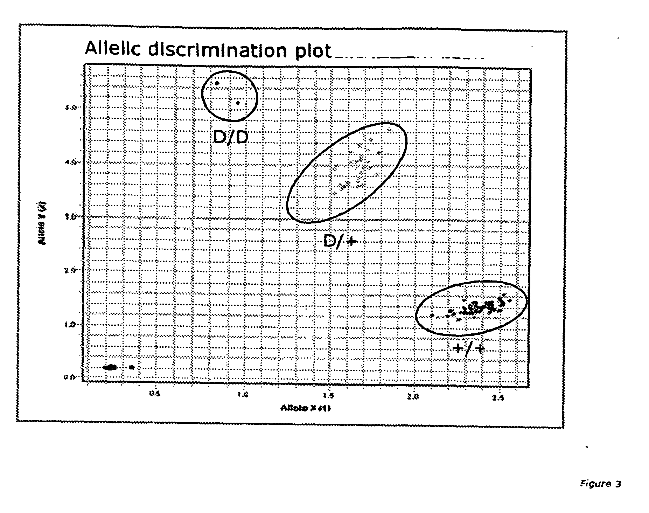

Figure 3 summarizes the results of the Taqman assay for genotyping of the 2bp deletion in the bovine *MRC2* gene. The graph illustrates allelic discrimination of the wild-type and the mutated alleles, allowing clear separation between the three groups: homozygous wild-types (+/+), carriers (D/+) and affected animals (D/D).

The present invention will be further described in more detail in the following examples, which however shall not be understood to limit the scope of the present invention in any way.

### Examples

### Example 1: Autozygosity mapping localized the CTS gene in a 2.42 Mb interval on BTA19.

In order to map putative susceptibility gene for CTS, samples from 10 affected individuals were collected. The samples were obtained from farms and diagnosed as CTS affected individuals. Ten healthy contemporary animals were included in the study in order to estimate allelic frequency at each SNP locus.

Genomic DNA was extracted from all samples and genotyped for a battery of 53000 autosomal SNPs (Single Nucleotide Polymorphismes). The present inventors searched for shared homozygous identical-by-descent (IBD) haplotypes amongst cases (autozygosity mapping). A 2.42 MB chromosomal segment, on BTA19, between the SNP markers [SNP_ULGR_BTA-45661] and [SNP_ULGR_AAFC03117912_21237], responded to those criteria.

### Example 2: A two base-pair deletion in the Mannose Receptor C type 2 gene coding for the Endocytic Transmembrane Glycoprotein Endo180 causes the CTS (Figure 2)

Comparative mapping allowed the identification of the HSA17q23.2 human genome segment orthologous to part of the corresponding bovine interval. Nineteen positional candidate genes were lying in this interval. The candidate genes were screened for mutation by re-sequencing coding exons from genomic DNA or cDNA and comparing sequences from affected individuals to wild-type sequences.

A two base pair deletion in exon 20 of the *Mannose Receptor C type 2* gene *(MRC2)* inducing a frame-shift in the open reading frame was identified which is resulting in a truncated protein which is about 30% shorter than the wild-type protein. The truncated protein is missing 3 C-type lectin domains and, more importantly, the transmembrane domain as well as the cytoplasmic domain. In consequence, this truncated protein will be unable to uptake and internalize the collagen or to play any receptor function.

During the studies for the present invention a 5'exonuclease assay was developed in order to facilitate genotyping of the mutation. During the studies fifty CTS cases were genotyped. As result it was found that all CTS cases were homozygous for the identified mutation as expected. Next, more than 3,000 healthy Belgian Blue bulls were genotyped. The *MRC2* c.2904_2905delAG mutation was found at a frequency of 25 % in the Belgian Blue bulls, with however no homyzygotes amongst unaffected individuals. Altogether these results allowed the incriminate of the Endo180 *L970fsX1002* mutation as being responsible for CTS.

**Example 3: Taqman assay for genotyping the *c.2904_2905delAG* mutation in the bovine (****Figure 3****)**

A genotyping test was developed using the TaqMan assay for allelic discrimination of SNPs, on an ABI7900HT instrument (Applied Biosystems, Foster City, CA). PCR amplification of the template DNA sequence was carried out with unlabeled forward and reverse primers (forward primer: 5'-GCG CAA CAG CAC CAG AGA-3', SEQ ID NO:9 and reverse primer: 5'-CTC CCT ACC TTG TTC AGG AAC TG-3', SEQ ID NO:10) following the recommendations of the manufacturer. The TaqMan probes had a non fluorescent quencher plus a minor groove binder attached to the 3' end and a reporter fluorescent label (Fam or Vic) attached to their 5' end. Each reaction mixture contained two probes having two nucleotide difference (mutated probe: 5'-CTG CCG CCC AC 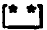 GGG-3', SEQ ID NO:7 and wild-type probe: 5'-CTG CCG CCC AC 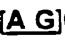 G-3', SEQ ID NO:8) and respectively Fam or Vic labels.

During strand replacement, fully hybridized probes remained bound, resulting in a cleavage of the reporter dye by the 5'-nuclease activity of Taq polymerase. The release of the reporter dyes correlated with the proportion of the matching alleles and resulted in a good discrimination between homozygous and heterozygous states.

### References

1. Strachan, T.; Read, A.P. (2004). Human Molecular Genetics 3. Garland Science.
2. Charlier C, Coppieters W, Rollin F, Desmecht D, Agerholm JS, Cambisano N, Carta E, Dardano S, Dive M, Fasquelle C, Frennet JC, Hanset R, Hubin X, Jorgensen C, Karim L, Kent M, Harvey K, Pearce BR, Simon P, Tamma N, Nie H, Vandeputte S, Lien S, Longeri M, Fredholm M, Harvey RJ, Georges M.Highly effective SNP-based association mapping and management of recessive defects in livestock.Nat Genet. 2008 Apr;40(4):449-54. Epub 2008 Mar 16.
3. Wienke D, MacFadyen JR, Isacke CM. Identification and characterization of the endocytic transmembrane glycoprotein Endo180 as a novel collagen receptor.Mol Biol Cell. 2003 Sep;14(9):3592-604. Epub 2003 Jul 25.
4. Behrendt N, Jensen ON, Engelholm LH, Mørtz E, Mann M, Danø K.A urokinase receptor-associated protein with specific collagen binding properties. J Biol Chem. 2000 Jan 21;275(3):1993-2002.
5. East L, Rushton S, Taylor ME, Isacke CM. Characterization of sugar binding by the mannose receptor family member, Endo180.J Biol Chem. 2002 Dec 27;277(52):50469-75. Epub 2002 Oct 23
6. Syvanen, A.C. (2001) Accessing genetic variation: genotyping single nucleotide polymorphisms. Nature Reviews Genetics 2: 930-942.

## Claims

1. Use of the Mannose Receptor C type 2 (*MRC2*) gene locus coding for the Endocytic Transmembrane Glycoprotein Endo180 protein (SEQ ID NO:5) as marker for detecting whether or not a bovine individual is carrier of Crooked Tail Syndrome (CTS) wherein a bovine individual having a mutated *MRC2* gene is judged to have carrier status for Crooked Tail Syndrome (CTS).

2. An in-vitro method for detecting whether or not a bovine individual is carrier of Crooked Tail Syndrome (CTS), by analyzing the *MRC2* gene locus coding for the Endo180 protein wherein a bovine individual having a mutated *MRC2* gene is judged to have carrier status for Crooked Tail Syndrome (CTS).

3. An assay for detecting whether or not a bovine individual is carrier of Crooked Tail Syndrome (CTS), wherein the *MRC2* gene locus coding for the Endo180 protein is analyzed wherein a bovine individual having a mutated *MRC2* gene is judged to have carrier status for Crooked Tail Syndrome (CTS).

4. The use, the method or the assay according to anyone of claims 1 to 3 wherein the mutation is rendering inactive or removing any one or more of the transmembrane domain, the cytoplasmic domain or one or more of the C-type lectin domains of the Endo180 protein.

5. The use, the method or the assay according to any one of claims 1 to 3, wherein a bovine individual having a mutated *MRC2* gene coding for a truncated Endo180 protein is judged to have carrier status for Crooked Tail Syndrome (CTS).

6. The use, the method or the assay according to claim 5, wherein the truncation is rendering inactive or removing any one or more of the transmembrane domain, the cytoplasmic domain or one or more of the C-type lectin domains of the Endo180 protein.

7. The use, the method or the assay according to any one of claims 1 to 6, wherein the presence of an allele of the *MRC2* sequence, which is **characterized by** a two base pair deletion at nucleotide positions 2904 and 2905 of the coding sequence in respect of the wild-type MRC2 genomic sequence shown in SEQ ID NO:1 or the wild-type cDNA sequence shown in SEQ ID NO:3 resulting in a frame-shift of the reading frame and in a truncation of the Endo180 protein, indicates carrier status.

8. The use, the method or the assay according to any one of claims 1 to 7, wherein the presence of an allele of the *MRC2* sequence, which is **characterized by** the nucleotides AG at nucleotide positions 2904 and 2905 of the coding sequence as shown in the wild-type MRC2 genomic sequence shown in SEQ ID NO:1 or as shown in in the wild-type cDNA sequence shown in SEQ ID NO:3 and corresponds to the wild-type Endo180 protein, indicates non-carrier status.

9. The use, the method or the assay according to any one of claims 1 to 8, wherein the genomic DNA is analyzed and wherein the presence of the sequence SEQ ID NO: 2 indicates carrier status.

10. The use, the method or the assay according to any one of claims 1 to 8, wherein the RNA is transcribed into cDNA by reverse transcription and analyzed and wherein the presence of the sequence SEQ ID NO:4 indicates carrier status.

11. The use, the method or the assay according to any one of claims 1 to 10, wherein the primer comprising the sequence 5'-CTG CCG CCC AC[**] GGG-3' (SEQ ID NO:7) or its complementary sequence is used for detecting the disease-causing allele of the *MRC2* gene and thus indicating the carrier status.

12. The use, the method or the assay according to any one of claims 1 to 11, wherein the primer comprising the sequence 5'-CTG CCG CCC AC**A G**G-3' (SEQ ID NO: 8) or its complementary sequence is used for detecting the wild-type allele of the *MRC2* gene and thus indicating the non-carrier status.

13. The use, the method or the assay according to any one of claims 1 to 12, wherein primers are used for the amplification of a sequence selected from SEQ ID NO: 1, SEQ ID NO: 2, SEQ ID NO: 3 and SEQ ID NO: 4 or a partial sequence thereof, wherein the partial sequence comprises at least the nucleotide positions 2904 and 2905 of the coding sequence.

14. The use, the method or the assay according to claim 13, wherein the amplification is carried out before the step of analyzing if or if not the disease-causing allele of MRC2 is present.

15. The use, the method or the assay according to any one of claims 1 to 14, wherein a bovine individual being homozygous in respect to the disease-causing allele in the *MRC2* gene locus is judged to have or to develop Crooked Tail Syndrome (CTS).

16. The use, the method or the assay according to any one of claims 1 to 15, wherein the sample can be any material containing nucleated cells from said animal including blood, sperm, hair, milk, tissues.

17. A reagent kit for detecting whether or not a bovine individual is carrier of Crooked Tail Syndrome (CTS), comprising
a) a primer comprising the sequence 5'-CTG CCG CCC AC[**] GGG-3' (SEQ ID NO: 7) or its complementary sequence for detecting the disease-causing allele of the *MRC2* gene and thus indicating the carrier status; and optionally
b) a primer comprising the sequence 5'-CTG CCG CCC AC**A G**G-3' (SEQ ID NO: 8) or its complementary sequence for detecting the wild-type allele of the *MRC2* gene and thus indicating the non-carrier status.

## Patentansprüche

1. Verwendung des Mannoserezeptor-C-Typ-2-(*MRC2*-) Genlocus, kodierend für das endolytische Transmembranglykoprotein Endo-180-Protein (SEQ ID NR: 5), als Marker zum Nachweis, ob ein Rind Träger des Crooked-Tail-Syndroms (CTS) ist oder nicht, wobei ein Rind mit einem mutierten *MRC2*-Gen als Träger des Crooked-Tail-Syndroms (CTS) betrachtet wird.

2. Ein In-vitro-Verfahren zum Nachweis, ob ein Rind Träger des Crooked-Tail-Syndroms (CTS) ist oder nicht, durch Analysieren des *MRC2-*Genlocus, der für das Endo-180-Protein kodiert, wobei ein Rind mit einem mutierten *MRC2*-Gen als Träger des Crooked-Tail-Syndroms (CTS) betrachtet wird.

3. Assay zum Nachweis, ob ein Rind Träger des Crooked-Tail-Syndroms (CTS) ist oder nicht, wobei der *MRC2*-Genlocus, der für das Endo-180-Protein kodiert, analysiert wird, wobei ein Rind mit einem mutierten *MRC2*-Gen als Träger des Crooked-Tail-Syndroms (CTS) betrachtet wird.

4. Verwendung, Verfahren oder Assay nach einem der Ansprüche 1 bis 3, wobei die Mutation eine oder mehrere aus der Transmembrandomäne, der cytoplasmatischen Domäne oder eine oder mehrere aus den C-Typ-Lektindomänen des Endo-180-Proteins inaktiviert oder beseitigt.

5. Verwendung, Verfahren oder Assay nach einem der Ansprüche 1 bis 3, wobei ein Rind mit einem mutierten *MRC2*-Gen, das für ein verkürztes Endo-180-Protein kodiert, als Träger des Crooked-Tail-Syndroms (CTS) betrachtet wird.

6. Verwendung, Verfahren oder Assay nach Anspruch 5, wobei die Verkürzung eine oder mehrere aus der Transmembrandomäne, der cytoplasmatischen Domäne oder eine oder mehrere aus den C-Typ-Lektindomänen des Endo-180-Proteins inaktiviert oder beseitigt.

7. Verwendung, Verfahren oder Assay nach einem der Ansprüche 1 bis 6, wobei das Vorliegen eines Allels der *MRC2*-Sequenz, das durch eine Deletion von zwei Basenpaaren an den Nukleotidpositionen 2904 und 2905 der kodierenden Sequenz im Vergleich zu der in SEQ ID NR: 1 dargestellten genomischen Wildtyp-MRC2-Sequenz oder der in SEQ ID NR: 3 dargestellten Wildtyp-cDNA-Sequenz **gekennzeichnet** ist, die zu einer Leserasterverschiebung des Leserasters und zu einer Verkürzung des Endo-180-Proteins führt, auf den Träger-Status hindeutet.

8. Verwendung, Verfahren oder Assay nach einem der Ansprüche 1 bis 7, wobei das Vorliegen eines Allels der *MRC2*-Sequenz, **gekennzeichnet durch** die Nukleotide AG an den Nukleotidpositionen 2904 und 2905 der kodierenden Sequenz, wie sie in der in SEQ ID NR: 1 dargestellten genomischen Wildtyp-MRC2-Sequenz oder in der in SEQ ID NR: 3 dargestellten Wildtyp-cDNA-Sequenz gezeigt ist und die dem Wildtyp-Endo-180-Protein entspricht, auf den Nichtträger-Status hindeutet.

9. Verwendung, Verfahren oder Assay nach einem der Ansprüche 1 bis 8, wobei die genomische DNA analysiert wird und wobei das Vorliegen der Sequenz SEQ ID NR: 2 auf den Träger-Status hindeutet.

10. Verwendung, Verfahren oder Assay nach einem der Ansprüche 1 bis 8, wobei die RNA durch reverse Transkription in cDNA transkribiert und analysiert wird, wobei das Vorliegen der Sequenz SEQ ID NR: 4 auf den Träger-Status hindeutet.

11. Verwendung, Verfahren oder Assay nach einem der Ansprüche 1 bis 10, wobei der Primer, der die Sequenz **5'-CTG CCG CCC AC** 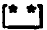 **GGG-3'** (SEQ ID NR: 7) oder ihre komplementäre Sequenz umfasst, zum Nachweis des krankheitsverursachenden Allels des *MRC2*-Gens verwendet wird und somit auf den Träger-Status hindeutet.

12. Verwendung, Verfahren oder Assay nach einem der Ansprüche 1 bis 11, wobei der Primer, der die Sequenz 5'-CTG CCG CCC AC**A G**G-3' (SEQ ID NR: 8) oder ihre komplementäre Sequenz umfasst, zum Nachweis des Wildtyp-Allels des *MRC2*-Gens verwendet wird und somit auf den Nichtträger-Status hindeutet.

13. Verwendung, Verfahren oder Assay nach einem der Ansprüche 1 bis 12, wobei Primer für die Amplifikation einer Sequenz verwendet werden, die aus SEQ ID NR: 1, SEQ ID NR: 2, SEQ ID NR: 3 und SEQ ID NR: 4 oder einer partiellen Sequenz davon ausgewählt sind, wobei die partielle Sequenz mindestens die Nukleotidpositionen 2904 und 2905 der kodierenden Sequenz umfasst.

14. Verwendung, Verfahren oder Assay nach Anspruch 13, wobei die Amplifikation vor dem Schritt der Analyse, ob das krankheitsverursachende Allel von MRC2 vorliegt oder nicht, durchgeführt wird.

15. Verwendung, Verfahren oder Assay nach einem der Ansprüche 1 bis 14, wobei für ein Rind, das homozygot für das krankheitsverursachende Allel am *MRC2*-Genlocus ist, derart beurteilt wird, dass es Crooked-Tail-Syndrom (CTS) hat oder entwickeln wird.

16. Verwendung, Verfahren oder Assay nach einem der Ansprüche 1 bis 15, wobei die Probe jedes beliebige Material, das Karyozyten aus dem Tier enthält, einschließlich Blut, Sperma, Haare, Milch, Gewebe, sein kann.

17. Ein Reagenskit zum Nachweisen, ob ein Rind Träger des Crooked-Tail-Syndroms (CTS) ist oder nicht, besteht aus:
a) einen Primer, der die Sequenz **5'-CTG CCG CCC AC** 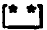 **GGG-3'** (SEQ ID NR: 7) oder ihre komplementäre Sequenz umfasst, zum Nachweisen des krankheitsverursachenden Allels des *MRC2*-Gens und somit zum Anzeigen des Träger-Status; und gegebenenfalls
b) einen Primer, der die Sequenz 5'-CTG CCG CCC AC**A G**G-3' (SEQ ID NR: 8) oder ihre komplementäre Sequenz umfasst, zum Nachweisen des Wildtyp-Allels des *MRC2*-Gens und somit zum Anzeigen des Nichtträger-Status.

## Revendications

1. Utilisation du locus du gène du récepteur du mannose des types C 2 (*MRC2*) codant pour la protéine Endo180 (Glycoprotéine Transmembranaire Endocytique) (SEQ ID NO : 5) comme marqueur pour détecter si un individu bovin est porteur ou non du syndrome de la queue tordue (SQT), dans laquelle un individu bovin ayant un gène *MRC2* muté est considéré comme ayant un statut de porteur du syndrome de la queue tordue (SQT).

2. Procédé *in vitro* pour détecter si un individu bovin est porteur ou non du syndrome de la queue tordue (SQT) en analysant le locus du gène *MRC2* qui code pour la protéine Endo180, dans lequel un individu bovin ayant un gène *MRC2* muté est considéré comme ayant un statut de porteur du syndrome de la queue tordue (SQT).

3. Un dosage pour détecter si un individu bovin est porteur ou non du syndrome de la queue tordue (SQT), dans lequel le locus du gène *MRC2* qui code pour la protéine Endo180 est analysé, dans lequel un individu bovin ayant un gène *MRC2* muté est considéré comme ayant un statut de porteur du syndrome de la queue tordue (SQT).

4. L'utilisation, le procédé ou le dosage selon l'une quelconque des revendications 1 à 3, dans lesquelles la mutation rend inactif ou supprime un quelconque ou plusieurs des domaine transmembranaire, domaine cytoplasmique ou un ou plusieurs des domaines lectine du type C de la protéine Endo180.

5. L'utilisation, le procédé ou le dosage selon l'une quelconque des revendications 1 à 3, dans lesquelles un individu bovin ayant un gène *MRC2* muté, qui code pour une protéine Endo180 tronquée, est considéré comme ayant un statut de porteur du syndrome de la queue tordue (SQT).

6. L'utilisation, le procédé ou le dosage selon la revendication 5, dans lesquelles la troncature rend inactif ou supprime un quelconque ou plusieurs des domaine transmembranaire, domaine cytoplasmique ou un ou plusieurs des domaines lectine du type C de la protéine Endo180.

7. l'utilisation, le procédé ou le dosage selon l'une quelconque des revendications 1 à 6, dans lesquelles la présence d'un allèle de la séquence *MRC2,* qui est **caractérisée par** une délétion de deux paires de bases aux positions des nucléotides 2904 et 2905 de la séquence codante par rapport à la séquence génomique du *MRC2* de type sauvage présentée à la SEQ ID NO : 1 ou à la séquence d'ADNc de type sauvage présentée à la SEQ ID NO : 3, qui conduit à un changement dans le cadre du cadre de lecture et à une troncature de la protéine Endo180, indique un statut de porteur.

8. l'utilisation, le procédé ou le dosage selon l'une quelconque des revendications 1 à 7, dans lesquelles la présence d'un allèle de la séquence *MRC2* qui est **caractérisé par** les nucléotides AG aux positions des nucléotides 2904 et 2905 de la séquence codante, tel que présenté dans la séquence génomique du *MRC2* de type sauvage présentée à la SEQ ID NO : 1 ou tel que présenté dans la séquence d'ADNc de type sauvage présentée à la SEQ ID NO : 3, et qui correspond à la protéine Endo180 de type sauvage, indique un statut de non porteur.

9. l'utilisation, le procédé ou le dosage selon l'une quelconque des revendications 1 à 8, dans lesquelles l'ADN génomique est analysé et dans lequel la présence de la séquence SEQ ID NO : 2 indique un statut de porteur.

10. l'utilisation, le procédé ou le dosage selon l'une quelconque des revendications 1 à 8, dans lesquelles l'ARN est transcrit en ADNc par transcription inverse et est analysé et dans lequel la présence de la séquence SEQ ID NO : 4 indique un statut de porteur.

11. L'utilisation, le procédé ou le dosage selon l'une quelconque des revendications 1 à 10, dans lesquelles l'amorce comprenant la séquence 5'-CTG CCG CCC AC 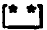 GGG-3'(SEQ ID NO : 7) ou sa séquence complémentaire est utilisée pour détecter l'allèle du gène *MRC2* causant la maladie et indiquer ainsi le statut de porteur.

12. L'utilisation, le procédé ou le dosage selon l'une quelconque des revendications 1 à 11, dans lesquelles l'amorce comprenant la séquence 5'-CTG CCG CCC AC**A G**G-3' (SEQ ID NO : 8) ou sa séquence complémentaire est utilisée pour détecter l'allèle de type sauvage du gène *MRC2* et indiquer ainsi le statut de non porteur.

13. L'utilisation, le procédé ou le dosage selon l'une quelconque des revendications 1 à 12, dans lesquelles des amorces sont utilisées pour amplifier une séquence choisie parmi les SEQ ID NO : 1, SEQ ID NO : 2, SEQ ID NO : 3 et SEQ ID NO : 4 ou une séquence partielle de celles-ci, dans lesquelles la séquence partielle comprend au moins les positions de nucléotides 2904 et 2905 de la séquence codante.

14. L'utilisation, le procédé ou le dosage selon la revendication 13, dans lesquelles l'amplification est réalisée avant l'étape qui analyse si l'allèle du *MRC2* causant la maladie est présent ou non.

15. L'utilisation, le procédé ou le dosage selon l'une quelconque des revendications 1 à 14, dans lesquelles un individu bovin étant homozygote pour l'allèle qui cause la maladie dans le locus du gène *MRC2* est considéré comme ayant le syndrome de la queue tordue (SQT) ou le développant.

16. L'utilisation, le procédé ou le dosage selon l'une quelconque des revendications 1 à 15, dans lesquelles l'échantillon peut être n'importe quel matériel contenant des cellules nucléées provenant dudit animal, y compris du sang, du sperme, du poil, du lait, des tissus.

17. Une trousse de réactifs pour détecter si un individu bovin est porteur ou non du syndrome de la queue tordue (SQT), comprenant :
a) une amorce, comprenant la séquence 5'-CTG CCG CCC AC 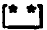 GGG-3' (SEQ ID n° : 7) ou sa séquence complémentaire destinée à détecter l'allèle du gène *MRC2* causant la maladie et indiquant ainsi le statut de porteur ; et, en option,
b) une amorce, comprenant la séquence 5'-CTG CCG CCC AC**A G**G-3' (SEQ ID n° : 8) ou sa séquence complémentaire destinée à détecter l'allèle de type sauvage du gène *MRC2* et indiquant ainsi le statut de non porteur.
